(19) Europäisches Patentamt
European Patent Office
Office européen des brevets

(11) Veröffentlichungsnummer: **0 018 588**
**B1**

(12) # EUROPÄISCHE PATENTSCHRIFT

(45) Veröffentlichungstag der Patentschrift: **29.12.82**

(51) Int. Cl.³: **C 07 C 125/073,** C 07 C 125/077, C 07 D 295/12 // C07D239/04

(21) Anmeldenummer: **80102185.8**

(22) Anmeldetag: **23.04.80**

(54) **Verfahren zur Herstellung von aliphatischen und cycloaliphatischen Di- und Polyurethanen.**

(30) Priorität: **30.04.79 DE 2917490**

(43) Veröffentlichungstag der Anmeldung: **12.11.80 Patentblatt 80/23**

(45) Bekanntmachung des Hinweises auf die Patenterteilung: **29.12.82 Patentblatt 82/52**

(84) Benannte Vertragsstaaten: **AT BE CH DE FR GB IT LI NL SE**

(56) Entgegenhaltungen: **US-A-2 806 051**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Towae, Friedrich, Dr., Parkstrasse 22, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Verfahren zur Herstellung von aliphatischen und cycloaliphatischen Di- und Polyurethanen

Die Erfindung betrifft ein Verfahren zur Herstellung von aliphatischen und/oder cycloaliphatischen Di- und/oder Polyurethanen aus primären aliphatischen und/oder cycloaliphatischen Di- und/oder Polyaminen und Carbamidsäureestern in Gegenwart von Alkoholen und gegebenenfalls Katalysatoren bei erhöhten Temperaturen.

N-substituierte Urethane werden technisch üblicherweise durch Umsetzung von Alkoholen mit Isocyanaten oder von Aminen mit Chlorkohlensäureester hergestellt, wobei sowohl die Isocyanate als auch die Chlorkohlensäureester durch Phosgenierung der entsprechenden Amine und Abspaltung von Chlorwasserstoff oder durch Phosgenierung der Alkohole gewonnen werden. (Methoden der organischen Chemie, Houben – Weyl, Band 8, Seiten 137, 120 und 101, Georg Thieme Verlag, Stuttgart, 1952.) Diese Verfahren sind technisch sehr aufwendig; ferner bringt die Verwendung von Phosgen wegen damit verbundener Sicherheits- und Umweltschutzmaßnahmen erhebliche Nachteile mit sich.

N-substituierte Urethane finden als Zwischen- und Endprodukte Verwendung. Gemäß DE-OS 26 35 490 oder US-PS 3 919 278 sind sie beispielsweise zur Herstellung von Isocyanaten geeignet. Andere Herstellungsverfahren für N-substituierte Urethane gewinnen daher zunehmend an Bedeutung.

So beschreibt die DE-OS 2 160 111 ein Verfahren zur Herstellung von N-substituierten Urethanen durch Umsetzung eines organischen Carbonats mit einem primären oder sekundären Amin in Gegenwart einer Lewissäure. Nachteilig an diesem Verfahren ist, daß die Reaktionsgeschwindigkeit recht gering und die Reaktionszeiten dementsprechend groß sind; außerdem werden als Nebenprodukte zusätzlich stets N-Alkyl-arylamine erhalten.

Nach Angaben der US-PS 2 834 799 werden Carbamidsäure- und Kohlensäureester durch Umsetzung von Harnstoffen mit Alkoholen in Gegenwart von Bortrifluorid hergestellt. Nachteilig ist hierbei, daß das Bortrifluorid als Katalysator in äquimolaren Mengen benötigt wird, so daß pro erzeugtes Molekül Carbamidsäureester mindestens ein und pro Molekül Kohlensäureester mindestens zwei Moleküle Bortrifluorid verbraucht werden. Dadurch gestaltet sich das Verfahren nicht nur teuer, sondern es stellt auch eine erhebliche Umweltbelstung dar, da das Bortrifluorid in Form des $H_3N \cdot BF_3$-Adduktes anfällt.

Methyl-N-phenylurethan kann ferner aus Kohlenmonoxid Schwefel, Anilin und Methanol hergestellt werden (R. A. Franz et al, J. Org. Chem. 28, 585 (1963)). Nachteilig sind hierbei insbesondere die geringen Ausbeuten, die auch bei langen Reaktionszeiten nicht über 25% liegen.

Nach Angaben der US-PS 2 409 712 können N-Alkyl- und N-Arylurethane durch Umsetzung von Monoaminen mit Harnstoff, N,N'-Dialkyl- oder N,N'-Diarylharnstoff und Alkoholen bei Temperaturen von 150° bis 350°C, gegebenenfalls unter erhöhtem Druck, hergestellt werden. Beispielhaft beschrieben wird jedoch lediglich die Herstellung von Alkylmonourethanen nach der genannten Methode, während die Herstellung von N,N'-disubstituierten Diurethanen und von Polyurethanen nicht erwähnt wird.

Zur Herstellung von N-substituierten Monourethanen wird der Harnstoff gemäß US-PS 2 677 698 zunächst mit Monoaminen in den entsprechenden N,N'-disubstituierten Harnstoff übergeführt, dieser wird gereinigt und anschließend mit Alkohol zur Reaktion gebracht.

Nachteilig an den genannten Verfahren sind neben einer aufwendigen Technik insbesondere die geringen Ausbeuten, die auch durch die Herstellung und Reinigung von N,N'-disubstituierten Harnstoffen nicht verbessert werden können.

Diese Nachteile können auch mit Hilfe des Verfahrens nach US-PS 2 806 051 nicht beseitigt werden. Nach Angaben dieser Patentschrift wird beispielsweise n-Hexylamin mit Harnstoff und Alkohol im Molverhältnis 1,0 : 1,2 : 2,0 bei Temperaturen unter 200°C, vorzugsweise von 120° bis 160°C umgesetzt. Auch in den vorzugsweise genannten Temperaturbereichen werden in technisch zweckmäßigen Reaktionszeiten nach dieser Verfahrensweise N-substituierte Urethane nur in geringen Ausbeuten erhalten. Es ist daher nicht überraschend, daß in der nachgängigen US-PS 3 076 007 zur Herstellung von N-Alkyl- und N-Cycloalkylurethanen die obengenannten Methoden unter Verwendung von gegebenenfalls substituierten Harnstoffen nicht beschrieben werden; erwähnt wird hingegen die Umsetzung von Phosgen mit Alkoholen zu Chloralkylformiaten und die anschließende Weiterreaktion mit Aminen zu Urethanen, während als besonders vorteilhaft zur Herstellung der Urethane die Reaktion von Aminen mit Äthylencarbonat empfohlen wird.

Es ist ferner bekannt, daß Carbamidsäureäthylester in siedendem Dioxan mit Aminen nicht reagieren (D. G. Crosby and C. Niemann J. Am. Chem. Soc. 76, 4458 (1954)) und daß N-Alkylurethane mit alkoholischer Ammoniaklösung bei Temperaturen von 160° bis 180°C eine alkalische Lösung ergeben, aus der nach Neutralisation mit Salzsäure Aminhydrochlorid, Harnstoff, Alkylharnstoff und Alkylurethan isoliert werden kann (M. Brander Rec. trav. chim 37 88 – 91 (1917).

Da bereits die Herstellung von N-monosubstituierten Carbamidsäureestern aus Monoaminen, Harnstoff und Alkoholen bzw. der Austausch der Aminogruppe in Carbamidsäureestern nur in mäßigen Ausbeuten gelingt, ist nicht überraschend, daß in keiner der genannten Patentschrift die Herstellung von Di- und/oder Polyurethanen, ausgehend von aliphatischen und/oder cycloaliphati-

schen Di- und/oder Polyaminen, nach diesen Verfahrensvarianten auch nur erwähnt wird.

Die Aufgabe der vorliegenden Erfindung bestand darin aliphatische und/oder cycloaliphatische Di- und/oder Polyurethane aus leicht zugänglichen Ausgangskomponenten, möglichst in einer Reaktionsstufe unter wirtschaftlich vertretbaren Bedingungen in guten Ausbeuten herzustellen. Die Verwendung von stark toxischen Ausgangsstoffen, beispielsweise von Phosgen, Kohlenmonoxid oder Katalysatoren, die toxisch sind oder im Laufe der Umsetzung toxische Verbindungen bilden, beispielsweise von Schwefelwasserstoff, sollte weitgehend vermieden werden.

Diese Aufgabe wurde gelöst durch ein Verfahren zur Herstellung von aliphatischen und und/oder cycloaliphatischen Di- und/oder Polyurethanen, das dadurch gekennzeichnet ist, daß man Carbamidsäureester der Formel $H_2N-COOR'$, in der R' einen gegebenenfalls substituierten aliphatischen Rest mit 1 bis 20 Kohlenstoffatomen, einen cycloaliphatischen Rest mit 3 bis 15 Kohlenstoffatomen oder einen aromatisch-aliphatischen Rest bedeutet, mit Aminen der Formel $R-(NH_2)_n$, in der R einen mehrwertigen, gegebenenfalls substituierten aliphatischen Rest mit 2 bis 20 Kohlenstoffatomen, einen cycloaliphatischen Rest mit 5 bis 12 Kohlenstoffatomen oder einen aliphatisch-cycloaliphatischen Rest mit 8 bis 50 Kohlenstoffatomen und n eine ganze Zahl von wenigstens 2 und nicht mehr als 6 bedeuten, in Gegenwart von gegebenenfalls substituierten primären und/oder sekundären aliphatischen Alkoholen und gegebenenfalls Katalysatoren bei erhöhten Temperaturen umsetzt und den entstehenden Ammoniak gegebenenfalls abtrennt.

Die Reaktion kann durch folgende Gleichung veranschaulicht werden:

$$R-(NH)_n + n\,H_2N-COOR' \xrightarrow{R'OH} R-(NHCOOR')_n + n\,NH_3$$

Die Bildung von aliphatischen und cycloaliphatischen Di- und/oder Polyurethanen, insbesondere in einem Verfahrensschritt und in guten Ausbeuten ist besonders überraschend, da nach bekannter Lehrmeinung aus Carbamidsäureestern oder Harnstoff und Diaminen die entsprechenden Diharnstoffe, beispielsweise aus Hexamethylen-diamin und Harnstoff Hexamethylen-diharnstoff, erhalten werden.

Aus Harnstoff und Alkohol können auch Urethane erhalten werden, die jedoch in Gegenwart von Aminen zu N,N'-disubstituierten Harnstoffen weiterreagieren. (Methoden der organischen Chemie, Houben–Weyl, Band 8, Seiten 151 und 140, Georg Thieme Verlag, Stuttgart, 1952) Anstelle von Diharnstoffen können ferner gemäß DE-PS 896 412 aus Diamiden der Kohlensäure, z. B. Harnstoff und Diaminen, deren Aminogruppen durch die Kette von mehr als 3 Atomen getrennt sind, hochmolekulare, verspinnbare Kondensationsprodukte hergestellt werden. Hochmolekulare Polyharnstoffe, beispielsweise mit Molekulargewichten von 8000 bis 10 000 und größer, werden auch erhalten, wenn man Diurethane mit Diaminen bei Temperaturen von ungefähr 150°C bis 300°C kondensiert (US-PS 2 181 663 und US-PS 2 568 885). Da Mono- und Polyurethane außerdem thermisch in Isocyanate, Alkohole und gegebenenfalls Olefine, Kohlendioxid, Harnstoff und Carbodiimide gespalten werden, wobei die erhaltenen Spaltprodukte wieder zahlreiche Folgeprodukte, z. B. Biurete, Allophanate, Isocyanurate, Polycarbodiimide u. a. bilden können (J. A. C. 80, 5495 (1958) und J. A. C. S. 78, 1946 (1956)), konnte nicht erwartet werden, daß unter sehr ähnlichen Reaktionsbedingungen nach dem erfindungsgemäßen Verfahren aliphatische und/oder cycloaliphatische Di- und/oder Polyurethane in guten Ausbeuten erhalten werden. Dies war besonders überraschend, da Versuche zur Herstellung von Diurethanen aus den obengenannten Produkten nach den erfindungsgemäßen Reaktionsbedingungen nicht zum Ziele führten.

Für die erfindungsgemäße Umsetzung mit Carbamidsäureestern in Gegenwart von Alkoholen eignen sich Amine der Formel $R-(NH_2)_n$, worin R einen mehrwertigen, gegebenenfalls substituierten aliphatischen oder cycloaliphatischen Rest oder gemischte Reste dieser Art und n eine ganze Zahl bedeuten, deren Wert der Valenzzahl von R entspricht und wenigstens 2, vorzugsweise 2 bis 5 und insbesondere 2 beträgt. Die aliphatischen Reste enthalten 2 bis 20, vorzugsweise 4 bis 16 und insbesondere 4 bis 12 Kohlenstoffatome; sie können eine geradkettige oder verzweigte Struktur besitzen und eingeschobene Heteroatome, z. B. Sauerstoff, Schwefel oder ein tertiäres Stickstoffatom, oder zweiwertige heterocyclische Reste als Brückenglieder gebunden enthalten. Die cycloaliphatischen Reste enthalten 5 bis 12, vorzugsweise 6 bis 12 Kohlenstoffatome, während die gemischten Reste dieser Art 8 bis 50, vorzugsweise 10 bis 15 Kohlenstoffatome aufweisen. Im einzelnen seien beispielhaft genannt:

aliphatische Diamine, wie Äthylendiamin, 1,3- und 1,2-Propylendiamin, 2,2-Dimethyl-1,3-propylendiamin, 1,4-Butylendiamin, 1,5-Pentamethylen-diamin, 1,6-Hexamethylen-diamin, 2,2,4-Trimethyl-, 1,6-hexamethylen-diamin, 1,8-Octamethylen-diamin, 1,10-Decylendiamin und 1,12-Dodecylen-diamin, cycloaliphatische Diamine, wie 1,2-, 1,3- und 1,4-Cyclohexan-diamin, 2,4- und 2,6-Hexahydrotoluylendiamin sowie die entsprechenden Isomerengemische, aliphatisch-cycloaliphatische Diamine, wie 1,4-Hexahydroxylylen-diamin, 4,4'-, 2,4'- und 2,2'-diamino-dicyclohexylmethan sowie die entsprechenden Isomerengemische 2,2-Di-(4-aminocyclohexyl)-propan, 3-Aminomethyl-3,5,5-trimethyl-cyclohexylamin und Dicyclopentadienylverbindungen der Formel

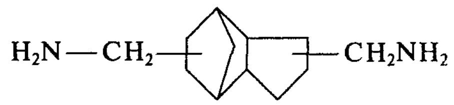

Polyamine, wie Polycyclohexyl-polymethylen-polyamine der Formel

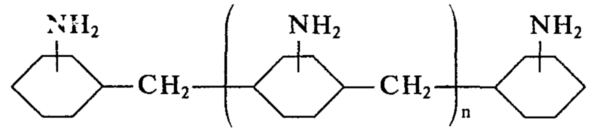

wobei n = 1 bis 4 bedeutet, und Mischungen aus Diamino-dicyclohexylmethanen und Polycyclohexyl-polymethylen-polyaminen und Heteroatome oder heterocyclische Reste gebunden enthaltende Diamine, wie 3,3'-Diamino-dipropyläther, gegebenenfalls substituierte N,N'-Bis-(aminoalkyl)-piperazine, z. B. N,N'-Bis-(2,2-dimethyl-3-aminopropyl)-piperazin und N,N'-Bis-(aminopropyl)-piperazin. Werden derartige Diurethane nach konventionellen Methoden, beispielsweise mit Phosgen oder Chlorkohlensäureester hergestellt, so fallen als Nebenprodukt beträchtliche Salzmengen an.

Besonders bewährt haben sich und daher vorzugsweise verwendet werden 1,6-Hexamethylen-diamin, 2,2,4-Trimethyl-1,6-hexamethylendiamin, 1,4-Hexahydroxylylen-diamin, 2,4- und 2,6-Hexahydrotoluylen-diamin sowie die entsprechenden Isomerengemische, 4,4'-Diamino-dicyclohexylmethan, 1,4-Diaminocyclohexan, 2,2-Di-(4-aminocyclohexyl)-propan und 3-Aminomethyl-3,5,5-trimethyl-cyclohexylamin.

Geeignete Carbamidsäureester besitzen die Formel $H_2N-COOR'$, in der R' einen gegebenenfalls substituierten aliphatischen, cycloaliphatischen oder aromatisch-aliphatischen Rest bedeutet. In Betracht kommen beispielsweise Carbamidsäureester auf Basis von primären aliphatischen Monoalkoholen mit 1 bis 20 Kohlenstoffatomen, vorzugsweise 1 bis 10 Kohlenstoffatomen, wie Carbamidäsure-methylester, -äthylester, -propylester, -n-butylester, -isobutylester, -2- und -3-methylbutylester, -neopentylester, -2-äthylbutylester, -2-methyl-pentylester, -n-hexylester, -2-äthylhexylester, -heptylester, -n-octylester, -n-nonylester, -n-decylester und -n-dodecylester, -2-phenylpropylester und -benzylester, auf Basis von sekundären aliphatischen und cycloaliphatischen Monoalkoholen mit 3 bis 15 Kohlenstoffatomen, vorzugsweise 3 bis 6 Kohlenstoffatomen, wie Carbamidsäure-isopropylester, -sek-butylester, -sek-iso-amylester, -cyclopentylester, -cyclohexylester, methyl-cyclohexylester und tert.-butyl-cyclohexylester. Vorzugsweise verwendet werden Carbamidsäure-methylester, -äthylester, -propylester, -butylester, -isobutylester, -2-äthylbutylester, -2- und -3-methylbutylester, -2-methylpentylester, -2-äthylhexylester, -heptylester, -octylester und -cyclohexylester.

Zur Herstellung der aliphatischen und/oder cycloaliphatischen Di- und/oder Polyurethane nach dem erfindungsgemäßen Verfahren werden die obengenannten aliphatischen, cycloaliphatischen und/oder aliphatisch-cycloaliphatischen Di- und/oder Polyamine mit den Carbamidsäureestern in solchen Mengen zur Reaktion gebracht, daß das Verhältnis von $NH_2$-Gruppen der Amine zu Carbamidsäureester 1 : 0,8 bis 10, vorzugsweise 1 : 0,9 bis 2,5 und insbesondere ungefähr 1 : 1 bis 2 beträgt.

Die Umsetzung wird in Gegenwart von Alkohol und in Abwesenheit oder Anwesenheit von Katalysatoren bei erhöhten Temperaturen und gegebenenfalls vermindertem oder erhöhtem Druck durchgeführt, wobei es sich als vorteilhaft erwiesen hat, den entstehenden Ammoniak sofort aus der Reaktionsmischung, beispielsweise durch Destillation, abzutrennen.

Als Alkohole für das erfindungsgemäße Verfahren können beliebige, gegebenenfalls substituierte primäre oder sekundäre aliphatische Alkohole sowie Mischungen davon verwendet werden. Vorzugsweise wird der den Carbamidsäureestern entsprechende Alkohol eingesetzt und zwar in einer Menge, daß das Verhältnis von $NH_2$-Gruppen der aliphatischen und/oder cycloaliphatischen Amine zu OH-Gruppen der Alkohole 1 : 0,25 bis 50, vorzugsweise 1 : 0,5 – 15 und insbesonders 1 : 0,5 – 7,5 beträgt.

In Betracht kommen beispielsweise primäre aliphatische Alkohole mit 1 bis 20 Kohlenstoffatomen, vorzugsweise 1 bis 10 Kohlenstoffatomen, wie Methanol, Äthanol, Propanol, 2-Phenyl-propanol, n-Butanol, Neopentylglykol, 2-Äthyl-butanol, 2-Methyl-pentanol, n-Hexanol, 2-Äthyl-hexanol, n-Heptanol, n-Octanol, n-Decanol und n-Dodecanol, Benzylalkohol, sekundäre aliphatische und cycloaliphatische Alkohole mit 3 bis 15 Kohlenstoffatomen, vorzugsweise 3 bis 6 Kohlenstoffatomen, wie Isopropanol, sec-Butanol, sec-Isoamylalkohol, Cyclopentanol, Cyclohexanol, Methylcyclohexanol und tert.-Butyl-cyclohexanol. Vorzugsweise verwendet werden als Monoalkohole Methanol, Äthanol, Propanol, n-Butanol, Isobutanol, n-Pentanol, 2-Äthyl-butanol, 2- und 3-Methylbutanol, 2-Methyl-pentanol, n-Hexanol, 2-Äthyl-hexanol, Heptanol, Octanol und Cyclohexanol.

Die Alkohole können gegebenenfalls mit anderen unter den Reaktionsbedingungen inerten organischen Lösungsmitteln gemischt werden.

Geeignet sind beispielsweise Lösungsmittelgemische, die mindestens 20 Gew.-%, vorzugsweise mehr als 50 Gew.-%, bezogen auf das Gesamtgewicht, Alkohol enthalten.

Die Herstellung der aliphatischen und/oder cycloaliphatischen Di- und/oder Polyurethane, vorzugsweise der Diurethane, wird erfindungsgemäß zweckmäßigerweise in Abwesenheit von Katalysatoren durchgeführt, da üblicherweise die Umsetzung in wirtschaftlich tragbaren Reaktionszeiten mit guten Ausbeuten erfolgt. Auf diese Weise werden kostspielige Reinigungsoperationen zur Abtrennung der Katalysatoren aus den erhaltenen Endprodukten vermieden.

Wird zur Erhöhung der Reaktionsgeschwindigkeit, vorzugsweise bei bei niedrigen Temperaturen, die Umsetzung jedoch in Gegenwart von Katalysatoren durchgeführt, so werden diese zweckmäßigerweise in Mengen von 0,1 bis 20 Gew.-%, vorzugsweise 0,5 bis 10 Gew.-% und insbesondere 1 bis 5 Gew.-%, bezogen auf das Gewicht des aliphatischen und/oder cycloaliphatischen primären Amins, verwendet.

Als Katalysatoren eignen sich anorganische oder organische Verbindungen, die ein oder mehrere, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäß Handbook of Chemistry and Physics 14th. Edition, publiziert von Chemical Rubber Publishing Co. 2310 Superior Ave. N. E. Cleveland, Ohio, enthalten, beispielsweise Halogenide, wie Chloride und Bromide, Sulfate, Phosphate, Nitrate, Borate, Hydroxide, Carboxylate, Chelate, Carbonate und Thio- oder Dithiocarbamate. Beispielhaft genannt seien die Kationen folgender Metalle: Lithium, Natrium, Kalium, Magnesium, Calcium, Aluminium, Gallium, Zinn, Blei, Wismut, Antimon, Kupfer, Silber, Gold, Zink, Quecksilber, Cer, Titan, Vanadium, Chrom, Molybdän, Mangan, Eisen, Kobalt und Nickel. Vorzugsweise Verwendung finden die Kationen von Lithium, Calcium, Aluminium, Zinn, Wismut, Antimon, Kupfer, Zink, Titan, Vanadium, Chrom, Molybdän, Mangan, Eisen und Kobalt. Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt: Lithiummethanolat, Lithiumäthanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminium-isobutylat, Aluminiumtrichlorid, Wismut-(III)-chlorid, Kupfer-(II)-acetat, Kupfer-(II)-sulfat, Kupfer-(II)-nitrat, Bis-(triphenylphosphinoxido)-kupfer-(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylenat, Cer-(IV)-oxid, Uranylacetat, Titan-tetrabutanolat, Titantetrachlorid, Titantetraphenolat, Titannaphthenat, Vanadium-(III)-chlorid, Vanadiumacetonylacetat, Chrom-(III)-chlorid, Molybdän-(VI)-oxid, Molybdänacetylacetonat, Wolfram-(VI)-oxid, Mangan-(II)-chlorid, Mangan-(II)-acetat, Mangan-(III)-acetat, Eisen-(II)-acetat, Eisen-(III)-acetat, Eisenphosphat, Eisenoxalat, Eisen-(III)-chlorid, Eisen-(III)-bromid, Kobaltacetat, Kobaltchlorid, Kobaltsulfat, Kobaltnaphthenat, Nickelchlorid, Nickelacetat und Nickelnaphthenat sowie deren Gemische.

Die Umsetzung wird bei Temperaturen von 160°C bis 300°C, vorzugsweise von 180° bis 250°C und insbesondere 185°C bis 230°C und Drücken von 0,1 bis 120 bar, vorzugsweise 0,5 bis 60 bar, insbesondere 1 bis 40 bar durchgeführt. Für diesen Temperaturbereich ergeben sich Reaktionszeiten von 0,1 bis 50 Stunden, vorzugsweise von 3 bis 20, insbesondere 5 bis 15 Stunden. Bei gegebener Temperatur wird die Reaktion dann vorzugsweise unter einem Druck durchgeführt, bei dem der entstehende Ammoniak selektiv aus dem Reaktionsgemisch abdestilliert werden kann. Die entsprechenden Werte können Tabellen mit physikalischen Kenndaten des Ammoniak und der Alkohole entnommen werden.

Nach dem erfindungsgemäßen Verfahren werden die Di- und/oder Polyurethane zweckmäßigerweise auf folgende Art hergestellt. Die primären aliphatischen und/oder cycloaliphatischen Di- und/oder Polyamine und die Carbamidsäureester werden in den genannten Molverhältnissen vorteilhafterweise in dem dem Carbamidsäureester entsprechenden Alkohol gelöst und in Abwesenheit oder gegebenenfalls Gegenwart von Katalysatoren in einem Reaktionsgefäß, ausgestattet mit einer Vorrichtung zur Abtrennung des Ammoniaks, erhitzt. Der entstehende Ammoniak kann nach beendeter Umsetzung abgetrennt werden; vorzugsweise wird er jedoch bereits im Laufe der Umsetzung abdestilliert. Hierbei kann es zweckmäßig sein, insbesondere bei der Umsetzung von molekularen Alkoholen unter Druck, den Ammoniak mit Hilfe eines unter den Reaktionsbedingungen inerten Strippmittels, beispielsweise eines Gases, wie Stickstoff, abzutrennen.

Nach einer besonders vorteilhaften Ausführungsform, die in der Regel zu einer erheblichen Reduzierung der Reaktionszeit und/oder einer beträchtlichen Erhöhung der Ausbeute führt, werden die primären aliphatischen und/oder cycloaliphatischen Di- und/oder Polyamine der Carbamidsäureester und der Alkohol zunächst in einem Verhältnis $NH_2$-Gruppen der Amine zu Carbamidsäureester zu Alkohol von 1 : 1—1,5 : 0,1—1, vorzugsweise 1 : 1—1,25 : 0,25—0,75, 1 bis 4, vorzugsweise 2 bis 3 Stunden lang zur Reaktion gebracht, danach wird der Reaktionsmischung zusätzlicher Alkohol in einer solchen Menge einverleibt, daß pro eingesetzter $NH_2$-Gruppe der Amine 1,5 bis 7,5, vorzugsweise 2 bis 5 Mol vorliegen, und die Reaktion in einem Zeitraum von insgesamt 4 bis 20 Stunden, vorzugsweise 5 bis 15 Stunden zu Ende geführt. Aus der erhaltenen Reaktionsmischung wird anschließend, gegebenenfalls nach Abtrennung des Katalysators, und/oder Abfiltrieren von Feststoffen das Di- und/oder Polyurethan isoliert, beispielsweise durch Abdestillieren des Alkohols und/oder des Lösungsmittels, sowie den gegebenenfalls im Überschuß eingesetzten O-Alkylcarbamaten, durch

partielles Abdestillieren des Alkohols und Auskristallisieren, durch Auskristallisieren, durch Ausfällen mit oder auch durch Umkristallisieren aus anderen Lösungsmitteln.

Die erhaltenen aliphatischen und/oder cycloaliphatischen Polyurethane sind wertvolle End- und Zwischenprodukte. Sie werden beispielsweise als Schädlingsbekämpfungsmittel verwendet. Als Zwischenprodukte werden sie als Komponenten für Polykondensations- und Polymersysteme eingesetzt, insbesondere jedoch unter Abspaltung von Alkohol in die entsprechenden Isocyanate übergeführt, wobei Di- und Polyisocyanate zur Herstellung von Polyurethanen Anwendung finden.

Die in den Beispielen genannten Teile beziehen sich auf das Gewicht. Die Elementarzusammensetzungen und Strukturen sind durch Elementaranalyse, Massenspektroskopie sowie IR- und NMR-Spektren bestätigt.

Beispiel 1

116 Teile 1,6-Hexamethylendiamin werden mit 346 Teilen O-Octylcarbamat und 2000 Teilen n-Octanol-(1) unter Abdestillieren von Ammoniak 16 Stunden bei Rückflußtemperatur (185—195° C) gerührt. Man filtriert aus dem auf 100—110° C abgekühlten Reaktionsgemisch den gebildeten Niederschlag ab, läßt das Reaktionsprodukt durch Abkühlen auf Raumtemperatur auskristallisieren und erhält durch Filtrieren, Waschen mit n-Octanol-(1) und Trocknen 236 Teile 1,6-Di-(octoxicarbonyl-amino)-hexan, $C_{24}H_{48}N_2O_4$ (Molekulargewicht 428) entsprechend 55% der Theorie, bezogen auf 1,6-Hexamethylendiamin und O-Octylcarbamat. Schmelzpunkt 106—108° C (aus Äthylacetat).

Beispiel 2

116 Teile 1,6-Hexamethylendiamin werden mit 346 Teilen O-Octylcarbamat, 3 Teilen Natrium-octylat und 1500 Teilen n-Octanol unter Abdestillieren von Ammoniak 12 Stunden bei Rückflußtemperatur (185—195° C) gerührt. Man filtriert den gebildeten Niederschlag aus dem auf 100—110° C abgekühlten Reaktionsgemisch ab, läßt das Reaktionsprodukt durch Abkühlen auf Raumtemperatur auskristallisieren und erhält durch Filtrieren, Waschen mit n-Octanol und Trocknen 242 Teile 1,6-Di-(octoxicarbonyl-amino)-hexan, entsprechend 56,5% der Theorie. Schmelzpunkt 106—107° C.

Beispiel 3

116 Teile 1,6-Hexamethylendiamin werden mit 380 Teilen O-Octylcarbamat und 130 Teilen n-Octanol-(1) unter Abdestillieren von Ammoniak 2 Stunden bei Rückflußtemperatur (185—205° C) gerührt. Dann läßt man zusätzlich 500 Teile n-Octanol-(1) in das Reaktionsgemisch einfließen und führt die Umsetzung 7 Stunden bei Rückflußtemperatur fort. Man filtriert das auf 110° C abgekühlte Reaktionsgemisch, läßt das Reaktionsprodukt durch Abkühlen auskristallisieren und erhält durch Filtrieren, Waschen mit n-Octanol-(1) und Trocknen 388 Teile 1,6-Di-(octoxi-carbonyl-amino)-hexan, entsprechend 90,6% der Theorie, bezogen auf 1,6-Hexamethylendiamin. Schmelzpunkt 107 bis 109° C.

Beispiel 4

232 Teile 1,6-Hexamethylendiamin werden mit 550 Teilen O-Butylcarbamat und 200 Teilen n-Butanol unter Durchsatz von 10 l Stickstoff pro Liter Reaktionsgemisch und Stunde über ein Tauchrohr 3 Stunden bei Rückflußtemperatur (185—195° C) und 6—7 bar gerührt. Dann läßt man zusätzlich 500 Teile n-Butanol in das Reaktionsgemisch einfließen und führt die Umsetzung 8 Stunden bei 195—200° C und ca. 7 bar fort. Man läßt das Produkt durch Abkühlen des Reaktionsgemisches auskristallisieren und erhält durch Filtrieren und Umkristallisieren aus Aceton/Wasser 563 Teile 1,6-Di-(butoxicarbonyl-amino)-hexan, $C_{16}H_{32}N_2O_4$ (Molekulargewicht 316), entsprechend 89% der Theorie, bezogen auf 1,6-Hexamethylendiamin. Schmelzpunkt 90—91° C.

Beispiel 5

232 Teile 1,6-Hexamethylendiamin werden mit 800 Teilen O-Äthylcarbamat und 120 Teilen Äthanol 2 Stunden bei Rückflußtemperatur (185—195° C) und 23—25 bar gerührt. Dann läßt man zusätzlich 500 Teile Äthanol in das Reaktionsgemisch einfließen und führt die Umsetzung unter Durchsatz von 8 l Stickstoff je Liter Reaktionsgemisch und Stunde bei 195—200° C 8 Stunden fort. Man engt das Reaktionsgemisch durch Abdestillieren von Äthanol weitgehend ein und kristallisiert das Produkt aus Aceton/Wasser um. Man erhält 432 Teile Di-(äthoxicarbonyl-amino)-hexan, $C_{12}H_{24}N_2O_4$ (Molekulargewicht 260), entsprechend 83% der Theorie, bezogen auf 1,6-Hexamethylendiamin. Schmelzpunkt 80—82° C.

Beispiel 6

116 Teile 1,6-Hexamethylendiamin werden mit 300 Teilen O-Cyclohexylcarbamat und 140 Teilen Cyclohexanol 2 Stunden bei Rückflußtemperatur (185—195°C) und 2—3 bar gerührt. Dann läßt man zusätzlich 600 Teile Cyclohexanol in das Reaktionsgemisch einfließen und führt die Umsetzung unter Durchsatz von 10 l Stickstoff je Liter Reaktionsgemisch und Stunde bei 195—200°C 8 Stunden fort. Man läßt das Reaktionsgemisch auf ca. 100°C abkühlen, filtriert und engt das Filtrat durch Destillation bis zu einer Sumpftemperatur von ca. 180°C bei 5—10 mbar ein. Durch Kristallisation des Rückstandes aus Methanol/Wasser erhält man 287 Teile 1,6-Di-(cyclohexoxicarbonyl-amino)-hexan, $C_{20}H_{36}N_2O_4$ (Molekulargewicht 368), entsprechend 78% der Theorie. Schmelzpunkt 98—100°C.

Beispiel 7

116 Teile 1,6-Hexamethylendiamin werden mit 360 Teilen 2-Butoxiäthoxi-carbamat und 118 Teilen 2-Butoxiäthanol-(1) unter Abdestillieren von Ammoniak 2 Stunden bei Rückflußtemperatur (180—200°C) gerührt. Dann läßt man zusätzlich 750 Teile 2-Butoxi-äthanol-(1) in das Reaktionsgemisch einfließen und führt die Umsetzung 7 Stunden bei Rückflußtemperatur fort. Man destilliert das nicht umgesetzte 2-Butoxiäthanol im Wasserstrahlvakuum weitgehend ab, kristallisiert den Rückstand aus Methanol/Wasser um und erhält durch Filtrieren und Trocknen 355 Teile 1,6-Di-(2-butoxy-äthoxycarbonyl-amino)-hexan, $C_{20}H_{40}N_2O_6$ (Molekulargewicht 404), entsprechend 87,8% der Theorie, bezogen auf 1,6-Hexamethylendiamin. Schmelzpunkt 64—65°C.

Beispiel 8

142 Teile 1,4-Hexahydroxylylen-diamin werden mit 380 Teilen O-Octylcarbamat und 150 Teilen n-Octanol-(1) unter Abdestillieren von Ammoniak 2 Stunden bei Rückflußtemperatur (185—200°C) gerührt. Dann läßt man zusätzlich 600 Teile n-Octanol in das Reaktionsgemisch einfließen und führt die Umsetzung 8 Stunden bei Rückflußtemperatur fort. Man filtriert das auf 110°C abgekühlte Reaktionsgemisch, läßt das Produkt durch Abkühlen des Filtrats auskristallisieren und erhält durch Filtrieren, Waschen mit Octanol-(1) und Trocknen 411 Teile 1,4-Di-(octoxycarbonyl-aminomethyl)-cyclohexan, $C_{26}H_{50}N_2O_4$ (Molekulargewicht 454), entsprechend 90,5% der Theorie, bezogen auf 1,4-Hexahydro-xylylendiamin. Schmelzpunkt 120—122°C (aus Äthylacetat).

Beispiel 9

210 Teile 4,4'-Diamino-dicyclohexyl-methan werden mit 360 Teilen O-Octylcarbamat und 150 Teilen n-Octanol-(1) unter Abdestillieren von Ammoniak 2 Stunden bei Rückflußtemperatur (185—205°C) gerührt. Dann läßt man zusätzlich 650 Teile n-Octanol in das Reaktionsgemisch einfließen und führt die Umsetzung 10 Stunden bei Rückflußtemperatur fort. Man filtriert das auf 100°C abgekühlte Reaktionsgemisch läßt das Produkt durch Abkühlen des Filtrats auskristallisieren und erhält durch Filtrieren, Waschen mit Octanol und Trocknen 468 Teile Di-(4-octoxi-carbonyl-aminocyclohexyl)-methan, $C_{31}H_{58}N_2O_4$ (Molekulargewicht 522), entsprechend 89,6% der Theorie, bezogen auf 4,4'-Diamino-dicyclohexyl-methan. Schmelzpunkt 129—130°C.

Beispiel 10

156 Teile Di-(3-aminopropyl)-äther werden mit 520 Teilen O-Octylcarbamat und 1000 Teilen n-Octanol-(1) unter Abdestillieren von Ammoniak 15 Stunden bei Rückflußtemperatur (185—200°C) gerührt. Man filtriert, destilliert nicht umgesetztes Octanol und O-Octylcarbamat bis zu einer Sumpftemperatur von 180—200°C bei ca. 2 mbar rasch ab und erhält einen beim Erkalten kristallisierenden Rückstand von 280 Teilen Di-(3-octoxicarbonyl-amino)-propyl-äther, $C_{24}H_{48}N_2O_5$ (Molekulargewicht 444), entsprechend 63% der Theorie, bezogen auf Di-(3-aminopropyl)-äther (Reinheit ca. 95%). Schmelzpunkt 61—62°C (aus Äthylacetat).

Beispiel 11

256 Teile N,N'-(Bis-2,2-dimethyl-3-aminopropyl)-piperazin werden mit 400 Teilen O-Octylcarbamat und 130 Teilen n-Octanol unter Abdestillieren von Ammoniak 1 Stunde bei Rückflußtemperatur (190—200°C) gerührt. Dann läßt man zusätzlich 650 Teile n-Octanol in das Reaktionsgemisch einfließen und führt die Umsetzung 7 Stunden bei Rückflußtemperatur fort. Man destilliert nicht umgesetztes

Octanol und O-Octylcarbamat bis zu einer Sumpftemperatur von 180—200°C bei 2 mbar und erhält durch Umkristallisieren des Rückstands aus Aceton/Wasser 506 Teile N,N'-Bis-(octoxycarbonyl-amino-neopentyl)-piperazin, $C_{32}H_{64}N_4O_4$ (Molekulargewicht 568), entsprechend 89% der Theorie, bezogen auf N,N'-Bis(2,2-dimethyl-3-aminopropyl)-piperazin. Schmelzpunkt 66—68°C.

## Beispiel 12

170 Teile 3-Aminomethyl-3,5,5-trimethyl-1-amino-cyclohexan werden mit 400 Teilen O-Octylcarbamat und 130 Teilen n-Octanol-(1) unter Abdestillieren des Ammoniaks 2 Stunden bei Rückflußtemperatur (185—200°C) gerührt. Dann läßt man zusätzlich 650 Teile n-Octanol-(1) in das Reaktionsgemisch einfließen und führt die Umsetzung 8 Stunden bei Rückflußtemperatur fort. Man filtriert, engt das Reaktionsgemisch bis zu einer Sumpftemperatur von ca. 200°C bei 2—3 mbar ein und erhält als partiell kristallisierenden Rückstand 448 Teile 3-(Octoxycarbonyl-amino)-methyl-3,5,5-trimethyl-1-(octoxycarbonylamino)-cyclohexan, $C_{28}H_{54}N_2O_4$ (Molekulargewicht 482), entsprechend 93% der Theorie, bezogen auf 3,5,5-Trimethyl-3-aminomethyl-1-amino-cyclohexan (Reinheit ca. 95%).

## Beispiel 13

102 Teile Diamino-neopentan werden mit 692 Teilen O-Octylcarbamat und 150 Teilen n-Octanol-(1) 2 Stunden bei 185—195°C gerührt. Dann läßt man zusätzlich 650 Teile n-Octanol in das Reaktionsgemisch einfließen und führt die Umsetzung 8 Stunden bei Rückflußtemperatur fort. Man destilliert aus dem Reaktionsgemisch n-Octanol und O-Octylcarbamat bis zu einer Sumpftemperatur von ca. 200°C bei 2—3 bar ab, nimmt den Rückstand in heißem Äthylacetat auf, filtriert das beim Abkühlen auskristallisierende 5,5-Dimethyl-hexahydropyrimidin-2-on ab und erhält nach Abdestillieren des Lösungsmittels aus dem Filtrat als Rückstand 178 Teile Di-(octoxicarbonyl-amino)-neopentan, $C_{23}H_{46}N_2O_4$ (Molekulargewicht 414) (Reinheit ca. 95%), entsprechend 43% der Theorie, bezogen auf Diamino-neopentan.

## Beispiel 14

5,8 Teile 1,6-Hexamethylendiamin werden mit 10,6 Teilen O-Äthylcarbamat und 9,2 Teilen Äthanol 12 Stunden lang bei 175°C gerührt, wobei über ein Druckventil im Reaktionsgefäß ein Druck von 15 bar eingestellt wird, so daß die Reaktionsmischung siedet. Der während der Reaktion gebildete Ammoniak wird unter Durchsatz von 7 Litern Stickstoff je Liter Reaktionsgemisch und Stunde kontinuierlich abdestilliert. Nach beendeter Reaktion wird die Mischung gaschromatographisch nach der Methode des »Internen Standard« analysiert. Hierbei zeigt sich, daß der Umsatz an 1,6-Hexamethylendiamin praktisch quantitativ ist und 9,9 Teile 1,6-Di-(äthoxycarbonylamino)-hexan (76,2% der Theorie, bezogen auf umgesetztes 1,6-Hexamethylendiamin) entstanden sind, was einer Raum-Zeit-Ausbeute von 32,2 g/l · h entspricht.

## Beispiele 15 bis 20

Man verfährt analog den Angaben von Beispiel 14, fügt der Reaktionsmischung jedoch zusätzlich 0,1 Teile eines Katalysators bei.

Die verwendeten Katalysatoren, die Reaktionszeiten und Ausbeuten sind in der Tabelle zusammengefaßt.

Tabelle

| Beispiele | Katalysator | Zeit<br>h | Ausbeute<br>% | Raum-Zeit-Ausbeute<br>g/l · h |
|---|---|---|---|---|
| 15 | Eisen(II)-acetat | 7 | 77,7 | 56,4 |
| 16 | Kobalt(II)-acetat | 10 | 91,5 | 46,5 |
| 17 | Zink(II)-acetat | 7 | 71,5 | 51,9 |
| 18 | Zinknaphthenat | 7 | 70,0 | 50,8 |
| 19 | Vanadiumtrichlorid | 5 | 64,6 | 65,6 |
| 20 | Mangan(II)-acetat | 5 | 58,5 | 59,4 |

**Patentansprüche**

1. Verfahren zur Herstellung von aliphatischen und/oder cycloaliphatischen Di- und/oder Polyurethanen, dadurch gekennzeichnet, daß man Carbamidsäureester der Formel $H_2N-COOR'$, in der R' einen gegebenenfalls substituierten aliphatischen Rest mit 1 bis 20 Kohlenstoffatomen, einen cycloaliphatischen Rest mit 3 bis 15 Kohlenstoffatomen oder einen aromatisch-aliphatischen Rest bedeutet, mit Aminen der Formel $R-(NH_2)_n$, in der R einen mehrwertigen, gegebenenfalls substituierten aliphatischen Rest mit 2 bis 20 Kohlenstoffatomen, einen cycloaliphatischen Rest mit 5 bis 12 Kohlenstoffatomen oder einen aliphatisch-cycloaliphatischen Rest mit 8 bis 50 Kohlenstoffatomen und n eine ganze Zahl von wenigstens 2 und nicht mehr als 6 bedeuten, in Gegenwart von gegebenenfalls substituierten primären und/oder sekundären aliphatischen Alkoholen und gegebenenfalls Katalysatoren bei erhöhten Temperaturen umsetzt und den entstehenden Ammoniak gegebenenfalls abtrennt.

2. Verfahren nach Anspruch 1, dadurch gekennzeichnet, daß das Verhältnis von $NH_2$-Gruppen der primären aliphatischen und/oder cycloaliphatischen Di- und/oder Polyamine zu Carbamidsäureester 1 : 0,8 bis 10 beträgt.

3. Verfahren nach Anspruch 1, dadurch gekennzeichnet, daß das Verhältnis von $NH_2$-Gruppen der primären aliphatischen und/oder cycloaliphatischen Di- und/oder Polyamine zu OH-Gruppen der Alkohole 1 : 0,25 bis 50 beträgt.

4. Verfahren nach Anspruch 1, dadurch gekennzeichnet, daß man die Umsetzung bei Temperaturen von 160° bis 300° C durchführt.

5. Verfahren gemäß Anspruch 1, dadurch gekennzeichnet, daß man den entstehenden Ammoniak gleichzeitig abtrennt.

6. Verfahren gemäß Anspruch 1, dadurch gekennzeichnet, daß man die Umsetzung bei Drücken von 0,1 bis 120 bar durchführt.

7. Verfahren gemäß Anspruch 1, dadurch gekennzeichnet, daß man als aliphatische Diamine 1,6-Hexamethylendiamin, als cycloaliphatische und aliphatisch-cycloaliphatische Diamine 1,4-Hexahydroxylylen-diamin, 2,4- und 2,6-Hexahydrotoluylendiamin sowie die entsprechenden Isomerengemische, 4,4'-Diaminodicyclohexyl-methan, 1,4-Diamino-cyclohexan, 2,2-Di-(4-aminocyclohexyl)-propan und 3-Aminomethyl-3,5,5-trimethylcyclohexylamin verwendet.

8. Verfahren gemäß Anspruch 1, dadurch gekennzeichnet, daß man als Carbamidsäureester solche aus Carbamidsäure und aliphatischen und cycloaliphatischen Monoalkoholen mit 1 bis 10 Kohlenstoffatomen im Alkoholrest verwendet.

9. Verfahren gemäß Anspruch 1, dadurch gekennzeichnet, daß man als Alkohole die den Carbamidsäureestern entsprechenden Alkohole verwendet.

10. Verfahren gemäß Anspruch 1, dadurch gekennzeichnet, daß man als Katalysatoren Verbindungen verwendet, die ein oder mehrere Kationen von Metallen aus den Gruppen IA, IB, IIA, IIB, IIIA, IIIB, IVA, IVB, VA, VB, VIB, VIIB und VIIIB des Periodensystems gebunden enthalten.

11. Verfahren gemäß Anspruch 1, dadurch gekennzeichnet, daß man die Ausgangskomponenten im Verhältnis $NH_2$-Gruppen der Amine zu Carbamidsäureester zu Alkohol von 1 : 1—1,5 : 0,1—1 1 bis 4 Stunden umsetzt, danach der Reaktionsmischung zusätzlichen Alkohol in einer Menge einverleibt, daß das Verhältnis $NH_2$-Gruppen der Amine zu Alkohol 1 : 1,5—7,5 beträgt und die Reaktion zu Ende führt.

## Claims

1. A process for the preparation of aliphatic and or cycloaliphatic di- and/or polyurethanes wherein carbamic esters of the formula $H_2N-COOR'$, where R' denotes an unsubstituted or substituted aliphatic radical having 1 to 20 carbon atoms, a cycloaliphatic radical having 3 to 15 carbon atoms or an aromatic-aliphatic radical, are reacted with amines of the formula $R-(NH_2)_n$, where N denotes a polyvalent unsubstituted or substituted aliphatic radical having 2 to 20 carbon atoms, a cycloaliphatic radical having 5 to 12 carbon atoms or an aliphatic-cycloaliphatic radical having 8 to 50 carbon atoms and n is a whole number of at least 2 and not more than 6 in the presence of unsubstituted or substituted primary and/or secondary aliphatic alcohols and, if desired, catalysts at elevated temperatures and, if desired, the ammonia that forms is separated.

2. The process of claim 1 wherein the ratio of $NH_2$ groups of the primary aliphatic and/or cycloaliphatic di- and/or polyamines to the carbamic esters is 1 : 0.8 to 10.

3. The process of claim 1 wherein the ratio of $NH_2$ groups of the primary aliphatic and/or cycloaliphatic di- and/or polyamines to the hydroxyl groups of the alcohols is 1 : 0.25 to 50.

4. The process of claim 1 wherein the reaction is carried out at temperatures of from 160° C to 300° C.

5. The process of claim 1 wherein the ammonia formed is simultaneously separated.

6. The process of claim 1 wherein the reaction is carried out at pressures of from 0.1 bar to 120 bars.

7. The process of claim 1 wherein the aliphatic diamine used is 1,6-hexamethylenediamine and the cycloaliphatic and aliphatic cycloaliphatic diamines used are 1,4-hexahydroxylenediamine, 2,4- and 2,6-hexahydrotoluenediamine, the corresponding isomer mixtures thereof, 4,4'-diamindicyclohexymethane, 1,4-diamincyclohexane, 2,2-bis(4-aminocyclohexyl)-propane and 3-aminomethyl-3,5,5-trimethylcyclohexylamine.

8. The process of claim 1 wherein the carbamic esters used are those of carbamic acid and aliphatic and cycloaliphatic monoalcohols having 1 to 10 carbon atoms in the alcohol radical.

9. The process of claim 1 wherein the alcohols used are the alcohols corresponding to the carbamic esters.

10. The process of claim 1 wherein the catalysts used are compounds containing, in bonded form, one or more cations of metals from the groups IA, IB, IIA, IIB, IIIA, IIIB, IVA, IVB, VA, VB, VIB, VIIB and VIIIB of the periodic system.

11. The process of claim 1 wherein the starting components are reacted in a ratio of $NH_2$ groups of the amines to carbamic esters to alcohol of 1 : 1—1.5 : 0.1—1 for one hour to four hours, then additional alcohol is added to the reaction mixture in an amount such that the ratio of $NH_2$ groups of the amines to alcohol is 1 : 1.5—7.5 and the reaction is completed.

## Revendications

1. Procédé de préparation de di- et(ou) de polyuréthannes aliphatiques et(ou) cycloaliphatiques, caractérisé en ce que l'on fait réagir des esters d'acide carbamique de la formule $H_2N-COOR'$, dans laquelle R' représente un groupe aliphatique en $C_1$ à $C_{20}$ éventuellement substitué, un groupe cycloaliphatique en $C_3$ à $C_{15}$ ou un reste aromatique-aliphatique, et des amines de la formule $R-(NH_2)_n$, dans laquelle R désigne un groupe aliphatique polyvalent en $C_2$ à $C_{20}$ éventuellement substitué, un groupe cycloaliphatique en $C_5$ à $C_{12}$ ou un reste aliphatique-cycloaliphatique en $C_8$ à $C_{50}$ et n est un nombre entier valant au moins 2, mais pas supérieur à 6, à des températures accrues en présence d'alcools aliphatiques primaires et (ou) secondaires éventuellement substitués et éventuellement de catalyseurs, l'ammoniac formé étant éventuellement séparé.

2. Procédé suivant la revendication 1, caractérisé en ce que la proportion des groupes-$NH_2$ des di- et(ou) polyamines aliphatiques et(ou) cycloaliphatiques primaires est à celle de l'ester de l'acide carbamique dans un rapport compris entre 1 : 0,8 et 1 : 10.

3. Procédé suivant la revendication 1, caractérisé en ce que la proportion des groupes-$NH_2$ des di- et(ou) polyamines aliphatiques et(ou) cycloaliphatiques primaires est à celle des groupes-OH des alcools dans un rapport compris entre 1 : 0,25 et 1 : 50.

4. Procédé suivant la revendication 1, caractérisé en ce que la réaction est effectuée à des températures de 160 à 300° C.

5. Procédé suivant la revendication 1, caractérisé en ce que l'ammoniac est séparé simultanément.

6. Procédé suivant la revendication 1, caractérisé en ce que la réaction est effectuée sous des pressions de 0,1 à 120 bars.

7. Procédé suivant la revendication 1, caractérisé en ce que l'on emploie comme diamine aliphatique la 1,6-hexaméthylène-diamine, comme diamines cycloaliphatiques et aliphatiques-cycloaliphatiques la 1,4-hexahydroxylylène-diamine, la 2,4- et 2,6-hexahydrotoluylène-diamine et les mélanges d'isomères de celles-ci, le 4,4'-diamino-dicyclohexyl-méthane, le 1,4-diamino-cyclohexane, le 2,2-di-(4-amino-cyclohexyl)-propane et la 3-aminométhyl-3,5,5-triméthyl-cyclohexyl-amine.

8. Procédé suivant la revendication 1, caractérisé en ce que les esters d'acide carbamique sont choises parmi ceux de l'acide carbamique et de monols aliphatiques et cycloaliphatiques avec une

fraction alcool en $C_1$ à $C_{10}$.

9. Procédé suivant la revendication 1, caractérisé en ce que l'alcool utilisé correspond à l'ester de l'acide carbamique.

10. Procédé suivant la revendication 1, caractérisé en ce que les catalyseurs sont choisis parmi les composés contenant un ou plusieurs cations de métaux des groupes IA, IB, IIA, IIB, IIIA, IIIB, IVA, IVB, VA, VB, VIB, VIIB et VIIIB du Système périodique des Eléments.

11. Procédé suivant la revendication 1, caractérisé en ce que l'on fait réagir les composants de départ dans un rapport groupes-$NH_2$ des amines/esters d'acide carbamique/alcool de 1 : 1—1,5 : 0,1—1 pendant 1 à 4 heures, on ajoute ensuite au mélange réactionnel une proportion supplémentaire d'alcool de manière à amener le rapport entre les groupes-$NH_2$ des amines et l'alcool entre 1 : 1,5 et 1 : 7,5, puis on complète la réaction.